(19) Europäisches Patentamt
European Patent Office
Office européen des brevets

(11) Publication number: **0 205 168**
**B1**

(12) **EUROPEAN PATENT SPECIFICATION**

(45) Date of publication of patent specification: **12.12.90**

(51) Int. Cl.$^5$: **C 07 D 519/04,** A 61 K 31/475

(21) Application number: **86107966.3**

(22) Date of filing: **12.06.86**

(54) **Furfuryl derivatives of vinblastine-type bis-indoles, a process for preparing same and pharmaceutical compositions containing them.**

(30) Priority: **12.06.85 HU 230185**

(43) Date of publication of application:
**17.12.86 Bulletin 86/51**

(45) Publication of the grant of the patent:
**12.12.90 Bulletin 90/50**

(84) Designated Contracting States:
**BE CH DE FR GB IT LI NL**

(56) References cited:
**DE-A-3 132 475**
**DE-A-3 132 476**

(73) Proprietor: **RICHTER GEDEON VEGYESZETI GYAR R.T.**
**Gyömröi ut 19-21**
**H-1475 Budapest X (HU)**

(72) Inventor: **Szántay, Csaba, Dr.**
**Zsombolyai ut 8**
**H-1113 Budapest (HU)**
Inventor: **Szabó, Lajos, Dr.**
**Visegrádi u. 3/b**
**H-1132 Budapest (HU)**
Inventor: **Honty, Katalin, Dr.**
**Gébics u. 4/a**
**H-1124 Budapest (HU)**
Inventor: **Keve, Tibor, Dr.**
**Hunyadlejtö 28**
**H-1124 Budapest (HU)**
Inventor: **Acs, Tibor**
**Szilárd u. 92**
**H-1174 Budapest (HU)**
Inventor: **Eckhardt, Sándor, Dr.**
**Julia u. 1**
**H-1026 Budapest (HU)**
Inventor: **Sugár, János, Dr.**
**Matróz u. 6**
**H-1051 Budapest (HU)**

**EP 0 205 168 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: Somfai, Zsuzsa, Dr.
Október 6. u. 16
H-1051 Budapest (HU)
Inventor: Iván, Eva, Dr.
Hajnóczi u. 6
H-1122 Budapest (HU)
Inventor: Kneffel, Zsuzsa, Dr.
Aitósi Dürer sor 7
H-1146 Budapest (HU)

(74) Representative: Beszédes, Stephan G., Dr.
Patentanwalt
Münchener Strasse 80a Postfach 1168
D-8060 Dachau (DE)

## Description

The invention is concerned with novel furfuryl derivatives of vinblastine-type, bis-indoles, a process for preparing same and pharmaceutical compositions, particularly having a cytostatic effect, containing them.

It is known that according to Hungarian patent specification Nos. 181,745 and 181,746 the O,$N_a$-acetals of vinblastine-type bis-indole alkaloids can be brought into an acetal-interchange reaction with appropriate nucleophilic reagents. The various S,$N_a$-acetals of bis-indoles have been prepared by using the same method (see EP—A—205 169).

The problem underlying to the invention is to create novel derivatives of vinblastine-type bis-indoles, a process for preparing same and pharmaceutical compositions, particularly having a cytostatic effect, containing them.

Surprisingly the above has been attained by the invention.

A subject-matter of the invention are furfuryl derivatives of vinblastine-type bis-indoles of the general formula

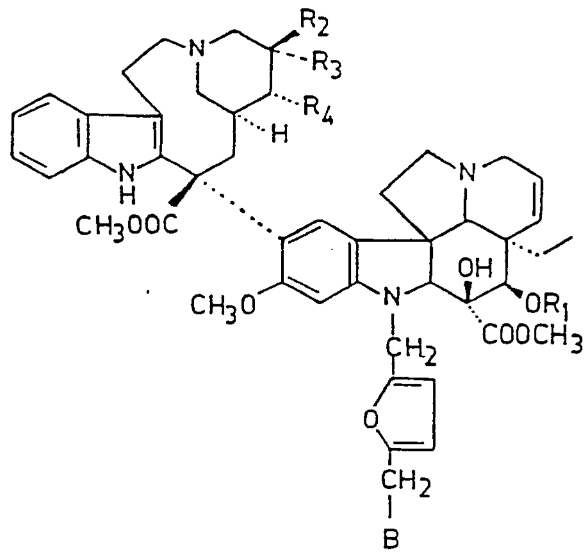

I,

wherein

$R_1$ stands for hydrogen atom or an acetyl group,

$R_2$ stands for a hydroxyl or ethyl group of β-position,

$R_3$ means an ethyl group of α-position,

$R_4$ represents a hydrogen or

$R_3$ and $R_4$ together represent an oxygen bridge and

B stands for a hydroxyl or an O-acyl group havinag 1 to 17 carbon atoms(s),

as well as their acid addition salts.

Preferably the acyl group which is represented by B is such having from 1 to 10, particularly 1 to 4, above all 1 to 2, carbon atom(s).

Particularly preferred compounds according to the invention are N-(demethyl)-N-(4-hydroxymethylfurfuryl)-vinblastine, N-(demethyl)-N-(4-hydroxymethylfurfuryl)-leurosine, N-(demethyl)-N-(4-acetoxymethylfurfuryl)-leurosine and 17-(deacetyl)-N-(demethyl)-N-(4-hydroxymethylfurfuryl)-vinblastine. Leurosine has the formula

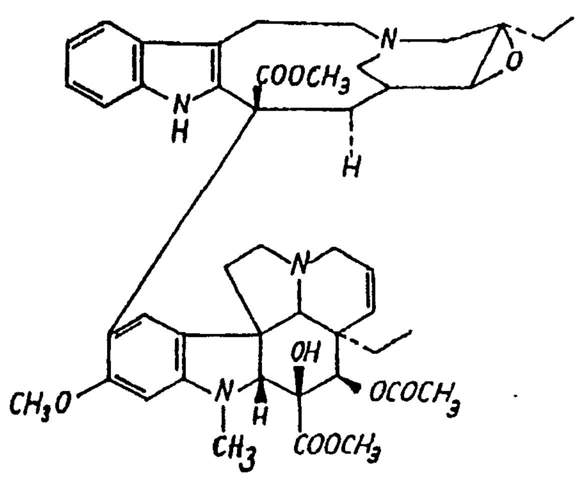

Now also it has been found that the said acetal-interchange reaction of the O,$N_a$-acetals of vinblastine-type bis-indole alkaloids with nucleophilic reagents takes a course different from the expected one when furfuryl alcohol is used as a nucleophilic reagent in the acetal-interchange reaction under suitably selected reaction conditions. Namely, according to the observations of the Applicant, when a furfuryl derivative of the general formula

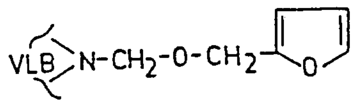

III

obtained from the compound of the general formula

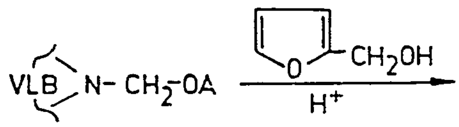


IIa

under mildly acidic reaction conditions, is reacted with an excess of furfuryl alcohol in a strongly acidic medium, then an α-aminomethylated furan derivative is obtained, wherein the $N_a$-nitrogen of the bis-indole dimer is presented as amino nitrogen. The compound of the general formula

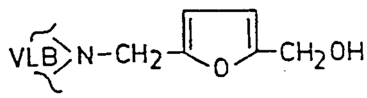

Ia

prepared in the same manner described above fall within the scope of the compounds of the general formula I. These reaction steps are illustrated in Reaction Scheme 1.

It has also been determined that the previous separation of the O,$N_a$-acetal formed with the furfuryl alcohol [e.g. separation of the compound of general formula III] is not necessary for obtaining the compounds of the general formula I. According to the Reaction Scheme 2, the desired furfuryl derivative may directly be prepared from any O,$N_a$- or S,$N_a$-acetal.

Reaction scheme 1

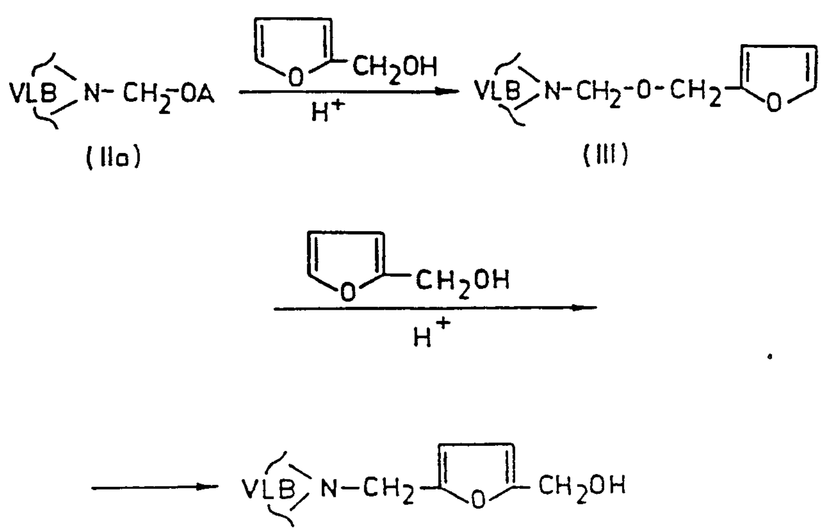


(IIa) (III)

(Ic)

Reaction scheme 2

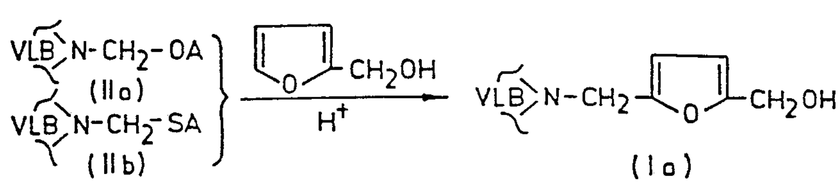


(IIa)

(IIb) (Ia)

Based on these facts, a subject-matter according to the invention is also a process for preparing the compounds according to the invention, which is characterized in that

a) a compound of the general formula

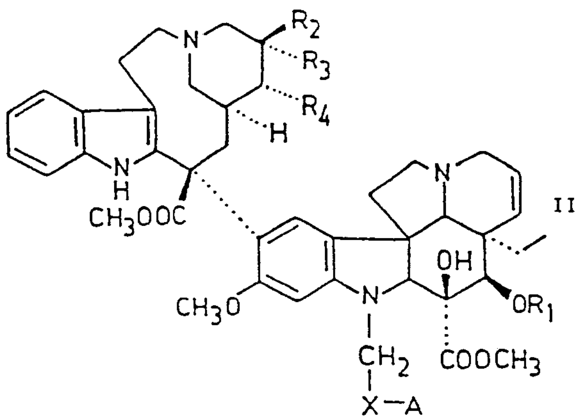

II,

wherein

$R_1$, $R_2$, $R_3$ and $R_4$ are the same as defined above

X means a sulphur or an oxygen atom;

and

A stands for an optionally substituted alkyl group having 1 to 10 carbon atom(s), phenylalkyl group having 1 to 10 carbon atom(s) in the alkyl part, cycloalkyl group having 3 to 8 carbon atoms, or a heteroaromatic group having 5 or 6 ring atoms,

is reacted with furfuryl alcohol in the presence of a mineral acid or a Lewis acid in a solvent, preferably in a halogenated hydrocarbon, then the reaction mixture is worked up and the desired product is isolated, and optionally

b) a compound of the general formula I, wherein

$R_1$ stands for an acetyl group, and

B represents a hydroxyl group

is transformed to a compound of formula I in which $R_1$ stands for a hydrogen atom or B represents an O-acyl group having from 1 to 17 carbon atom(s), respectively, by using a simple chemical operation, preferably a deacethylation or acylation, thus changing the meaning of $R_1$ and B, and then the thus obtained product is isolated and, if desired, the thus obtained product is transformed to an acid addition salt thereof.

According to a preferred embodiment of the process of the invention, the compounds of the general formula I are prepared as follows:

On carrying out the reaction a) of the process according to the invention the starting compound of the general formula II is dissolved in a solvent. Suitable solvents are ethers, ketones, benzene or its homologues as well as dimethyl formamide. Chlorinated hydrocarbons such as dichloromethane, chloroform and carbon tetrachloride are the most preferred solvents.

As catalyst a mineral acid, preferably hydrochloric or sulphuric acid, or Lewis acids may be used in this reaction. It is suitable to adjust the pH of the reaction mixture to a value lower than 3.

The process of the invention is carried out most preferably at room temperature, but the reaction may in general be realized at a temperature between −20°C and +20°C.

On carrying out reaction b) of the process according to the invention a compound of the general formula I is used as starting material which contains an acetyl group in 17-position or in which B represents a hydroxyl group. Namely, in the latter case, the terminal hydroxyl group permits the possibility of further transformations (e.g. acylation).

After carrying out the above reactions, the product may be separted from the reaction muxtre by means of extraction and/or evaporation and optionally purified by using a chromatographic method and/or recrystallization. The chromatography may be performed on a silica gel column.

The compounds used as starting materials in reaction a) of the process according to the invention are known. Derivatives, of N-demethyl-N-alkoxymethyl-vinblastine and -luerosine are described in the Hungarian patent specification No. 181,745; the thio derivatives are reported in EP—A—205 169).

In the starting materials of process a) preferably the alkyl group for which A may stand is preferably such having 1 to 4, most particularly 1 or 2, carbon atom(s). The phenylalkyl group preferably has 1 to 4, most particularly 1 or 2, carbon atom(s) in the alkyl part. The cycloalkyl group particularly has 4 to 7 and most particularly 5 or 6 carbon atoms. The heteroaromatic group having 5 or 6 ring atoms, preferably has 1 or more nitrogen, oxygen and/or sulphur atom(s) as [a] heteroatom(s). In case it has more than 1 heteroatom the number of them is preferably 2 or 3. Examples of such heteroaromatic groups are furyl, thienyl, pyrryl, coumaryl, thiocoumaryl, indolyl, oxazolyl, isooxazolyl, thiazolyl, imidazolyl, pyrazolyl, pyranyl, thiopyranyl, pyridyl, chinolyl and triazyl groups. The optional substituent(s) of the groups for which A may stand are preferably 1 or more acyloxy group(s) having from 1 to 4, particularly 1 or 2, carbon atom(s), alkoxy group(s); having from 1 to 4, particularly 1 or 2, carbon atom(s), nitro group(s), alkyl group(s) having from 1 to 4, particularly 1 or 2, carbon atom(s), aryl group(s), particularly phenyl group(s), aralkyl group(s), particularly phenylalkyl group(s), having from 1 to 4, particularly 1 or 2, carbon atom(s) in the alkyl part, cycloalkyl group(s) having from 3 to 8, particularly 4 to 7, most particularly 5 or 6, carbon atoms and/or heteroaromatic group(s) having 5 or 6 ring atoms and 1 or more nitrogen, oxygen and/or sulphur atom(s) as [a] heteroatom(s) examples of the latter being those above enumerated.

The compounds used as starting materials in reaction b) of the process according to the invention are novel and they are prepared by the reaction a) of the process according to the invention.

A subject-matter of the invention are also pharmaceutical compositions, characterized by a content of 1 or more compound(s) according to the invention as [an] active principle(s), suitably in admixture with 1 or more usual pharmaceutical carrier(s) and/or additive(s).

The compounds of the general formula I show a cytostatic activity with less toxicity than that of the known vinblastine-type bis-indole alkaloid drugs which are commercially available.

For investigating the biological activity, the injectable solutions containing water-insoluble compounds to be tested were dissolved by using physiological saline solution and one drop of Tween-80® each. These solutions were intraperitoneally administered in a volume of 0.1 ml/10 g of body-weight.

The effect of the novel compounds on intraperitoneally transplantable tumours (P388 mouse leukaemia) is reported hereinafter.

The P388 leukaemia was maintained in DBA/2 inbred mice and transplanted intraperitoneally by administering $10^6$ tumour cells/animal to groups consisting of 6 $BDF_1$ hybride mice each. In the 24th hour following the transplantation, the daily intraperitoneal treatment with the novel compounds was started. The body-weight and condition of the animals were daily controlled. The effect achieved on the animals treated for 8 days was expressed as the percentage of the mean survival time in days of the control group (T/C %).

TABLE

| Compound | Effect T/C % + | | | | | | |
|---|---|---|---|---|---|---|---|
| | 0.1 | 0.2 | 0.4 | 1.0 | 2.0 | 4.0 | 8.0 |
| | mg/kg | | | | | | |
| N-Demethyl-N-(4-hydroxymethyl-furfuryl)-vinblastine | — | — | 146 | 187 | 198 | 228 | 202 |
| 17-Deacetyl-N-demethyl-N-(4-hydroxy-methylfurfuryl)-vinblastine | — | — | 132 | 133 | 141 | 181 | 186 |
| Vineristine | 203 | 186 | 207 | (0.6 is toxic) | | | |

+ The effect is expressed as the percentage of the mean survival time of the control group

It is obvious from the Table that the compounds investigated are capable to significantly extend the life span of P388 leukaemic mice within a defined dose range.

The most important advantage of the compounds of the invention is that in the course of and after 8 times repeated administration of the effective doses, the paralyses of the hind limbs and of the bladder, which are observed on using vincristine and indicate neurotoxic effects, do not appear.

The invention is illustrated in detail by the aid of the following non-limiting Examples in which the percents indicated regarding the chromatographies are in volume.

Example 1

Preparation of N-demethyl-N-(4-hydroxymethylfurfuryl)-vinblastine

A) Preparation of N-demethyl-N-furfuryloxymethylvinblastine

1.5 ml (17 mmoles, about 50 equivalents) of furfuryl alcohol are added to a solution containing 300 mg (0.36 mmole) of N-demethyl-N-methoxymethylvinblastine in 40 ml of abs. dichloromethane at 0°C, then the pH value of the mixture is acidified to 5 to 6 by slowly adding abs. ethereal hydrogen chloride solution. The course of the reaction is followed by thin layer chromatography (TLC) (by using a mixture of developing system of dichloromethane and methanol in a volume ratio of 20:1.5; the $R_f$ value of the product is higher than that of the starting substance). At the end point of the reaction the acidic solution is neutralized by adding saturated potassium carbonate solution (2 to 3 ml), the organic layer is washed twice with 5 ml of water each, dried and evaporated under reduced pressure. The residue is triturated twice with 5 ml of petroleum ether each and purified by column chromatography under the following conditions.

Absorbent: silica gel with a particle size of 0.04—0.063 mm.

Column: 20 mm in diameter, 190 mm in height; in dichloromethane.

Dissolving and washing: dichloromethane, 10 + 50 ml.

Development: with 100 ml of a mixture containing 1% of methanol in dichloromethane.

Elution: with 600 ml of a mixture containing 3% of methanol in dichloromethane.

TLC: with a solvent system containing a mixture of methanol and dichloromethane in a volume ratio of 2:20. The repeated purification of the product may be achieved on $Al_2O_3$ II—III adsorbent by using dichloromethane and dichloromethane containing 1% of methanol, respectively. Thus, the aimed product is obtained in a yield of 150 mg (46%), m.p.: 218—221°C (amorphous);

$[\alpha]_D = +38°$ (c = 1, chloroform).

$C_{51}H_{62}N_4O_{11}$ (molecular weight: 906).

IR (KBr, $cm^{-1}$): 740, 930, 1040, 1220—1260, 1380, 1470, 1505, 1620, 1730, 2950, 3450.

B) Transformation of N-demethyl-N-furfuryloxymethylvinblastine to N-demethyl-N-(4-hydroxymethylfurfuryl)-vinblastine

30 µl of furfuryl alcohol are added to a solution containing 10 mg of N-demethyl-furfuryloxymethylvinblastine in 2 ml of abs. dichloromethane at room temperature, then the pH value is adjusted to 3 by adding abs. ethereal hydrogen chloride solution. According to the TLC analysis, the reaction proceeds instantaneously. Under anhydrous conditions, the thus-obtained product is free from side-products. After the common working-up by column chromatography 6 mg of the aimed product are obtained, m.p.: 190—194°C (amorphous);

$[\alpha]_D = -4°$ (c = 1, chloroform).

$C_{51}H_{62}N_4O_{11}$ (molecular weight 906)

IR (KBr, $cm^{-1}$): 740, 1030, 1010, 1200—1260;, 1375, 1460, 1500, 1510, 1730, 2950, 3400.

Example 2

Preparation of N-demethyl-N-(4-hydroxymethylfurfuryl)-vinblastine

2.55 ml (29 mmoles, 30 equivalents) of furfuryl alcohol are added to a solution containing 830 mg (0.98 mmole) of N-demethyl-N-(methoxymethyl)-vinblastine in 65 ml of abs. dichloromethane, then the pH value of the solution is adjusted to about 3 by adding abs. ethereal hydrogen chloride solution. The course of the reaction is followed by TLC (by using a developing system of a mixture of dichloromethane and methanol in a volume ratio of 20:2; the $R_f$ value of the product is lower than that of the starting material). At the end point of the reaction the acidic solution is neutralized by adding saturated potassium carbonate solution (2 to 3 ml), the mixture is diluted with water and after separating the phases, the aqueous layer is extracted 3 times with 20 ml of dichloromethane each. The combined organic phase is washed 3 times with 20 ml of water each, dried over anhydrous magnesium sulphate and evaporated under reduced pressure. The excess of the reagent is removed from the crude product by triturating 3 times with 15 ml of petroleum ether each and the oily residue is purified by column chromatography under the following conditions:

Adsorbent: silica gel with a particle size of 0.04—0.063 mm.

Column: 20 mm in diameter, 190 mm in height; in dichloromethane.

Dissolving and washing: dichloromethane, 10 + 100 ml.

Development: with 200 ml of a mixture containing 0.5% of methanol in dichloromethane.

Elution: with 200 ml of a mixture containing 1% of methanol in dichloromethane, then with 400 ml of a mixture containing 3% of methanol in dichloromethane; and finally with 400 ml of a mixture containing 5% of methanol in dichloromethane.

TLC: with a solvent system consisting of a mixture of methanol and dichloromethane in a volume ratio of 2:20.

By using the above process, 680 mg of the aimed product are obtained, the physical properties of which are in accordance with those of the compound prepared in Example 1.

Example 3

Preparation of N-demethyl-N-(4-hydroxymethylfurfuryl)-vinblastine hydrochloride

A solution containing 20 mg of N-demethyl-N-(4-hydroxymethylfurfuryl)-vinblastine in 1 ml of dichloromethane is acidified by adding abs. ethereal hydrogen chloride solution. The hydrochloride is precipitated by adding 2 ml of a mixture of abs. ether and petroleum in a volume ratio of 1:1. The thus obtained substance is amorphous without any characteristic melting point.

Example 4

Preparation of N-demethyl-N-(4-hydroxymethylfurfuryl)-leurosine

A solution containing 0.5 g (0.6 mmoles) of N-demethyl-N-methoxymethylleurosine and 1.3 ml (0.1 mmole, 25 equivalents) of furfuryl alcohol in 30 ml of abs. dichloromethane is acidified to pH 3 by adding abs. ethereal hydrogen chloride solution. The course of the reaction is followed by TLC (by using a developing system of a mixture of dichloromethane and methanol in a volume ratio of 20:2; the $R_f$ value of the starting material is higher than that of the product). At the end point of the reaction the acidic solution is neutralized by adding saturated potassium carbonate solution. The solution is diluted with water and after separation of the phases, the aqueous layer is extracted three times with 20 ml of dichloromethane each. The combined organic phase is washed with water, dried over anhydrous magnesium sulphate and evaporated under reduced pressure. The thus-obtained crude product is purified by column chromatography under the following conditions:

Absorbent: silica gel with a particle size of 0.063—0.2 mm.

Column: 20 mm in diameter, 150 mm in height; in dichloromethane.

Dissolving and washing: dichloromethane, 20 + 50 ml.

Development: with 200 ml of a mixture containing 0.5% of methanol in dichloromethane.

Elution: with 200 ml of a mixture containing 1% of methanol in dichloromethane; with 400 ml of a mixture containing 3% of methanol in a dichloromethane; and with 300 ml of a mixture containing 5% of methanol in dichloromethane.

TLC: with a solvent system containing a mixture of methanol and dichloromethane in a volume ratio of 2:20.

By using the above process, 399 mg (74%) of the aimed product are obtained.

IR (KBr, $cm^{-1}$): 730, 1020, 1200—1240, 1320, 1360, 1450, 1495, 1605, 1730, 2900, 3400.

Example 5

Preparation of N-demethyl-N-(4-hydroxymethylfurfuryl)-vinblastine from N-demethyl-N-(2-acetoxyethylthiomethyl)-vinblastine

250 µl (2.5 mmoles, 40 equivalents) of furfuryl alcohol are added to a solution containing 60 mg (µmoles) of the starting material in 50 ml of abs. dichloromethane. The solution is cooled to 0°C and acidified to pH 2 by adding abs. ethereal hydrogen chloride solution. The course of the reaction is followed by TLC (by using a developing system of a mixture of dichloromethane and methanol in a volume ratio of 20:2; the $R_f$ value of the starting material is higher than that of the product). At the end point of the reaction the acidic solution is neutralized by adding potassium carbonate solution. The solution is washed twice with 5 ml of water each, dried over anhydrous magnesium sulphate and evaporated under reduced pressure. The thus-obtained product is purified by column chromatography under the following conditions:

Adsorbent: silica gel with a particle size of 0.04—0.063 mm.

Column: 12 mm in diameter, 140 mm in height; in dichloromethane.

Dissolving and washing: dichloromethane, 10 + 50 ml.

Development: with 100 ml of a mixture containing 1% methanol in dichloromethane;

Elution: with 100 ml of a mixture containing 3% of methanol in dichloromethane; with 200 ml of a mixture containing 5% of methanol in dichloromethane.

TLC: with a solvent system containing a mixture of methanol and dichloromethane in a volume ratio of 2:20.

By using the above process, 40 mg (74%) of the aimed product are obtained, the physical properties of which are in agreement with those of the compound prepared in Example 1.

Example 6

Preparation of N-demethyl-N-(4-acetoxymethylfurfuryl)-leurosine

A solution containing 200 mg (0.23 mmole) of N-demethyl-N-(4-hydroxymethylfurfuryl)-leurosine, 480 mg (4 mmoles, 20 equivalents) of 4-dimethylaminopyridine and 1.2 ml (50 equivalents) of acetic anhydride in 20 ml of abs. dichloromethane is let to stand for 3 hours at room temperature. The course of the reaction is followed by TLC (by using a developing system of a mixture of dichloromethane and methanol in a

volume ratio of 20:2; the $R_f$ value of the product is higher than that of the starting substance). At the end point of the reaction, the solution is diluted with 30 ml of dichloromethane and 20 ml of water and alkalized to pH 9 by adding concentrated ammonium hydroxide solution. After separating the phases the organic layer is washed three times with 15 ml of water each, dried over anhydrous magnesium sulphate and evaporated under reduced pressure. The product is made free from the excess reagent by column chromatography to give the aimed product in a yield of 0.16 g (70%).

Example 7

Preparation of 17-deacetyl-N-demethyl-N-(4-hydroxymethylfurfuryl)-vinblastine

250 mg (0.28 mmole) of N-demethyl-N-(4-hydroxymethylfurfuryl)-vinblastine are dissolved in 10 ml of 0.5N sodium methoxide solution and set aside at room temperature for 8 hours. (The course of the reaction is followed by TLC by using a developing system of a mixture of dichloromethane and methanol in a volume ratio of 20:2; the $R_f$ value of the starting material is higher than that of the product). At the end point of the reaction, the base is neutralized by adding 0.3 ml (50 mmoles) of glacial acetic acid and the solution is evaporated under reduced pressure. The residue is dissolved in 40 ml of dichloromethane, washed twice with 15 ml of water each, dried and again evaporated. In this way 213 mg of a crude product are obtained which is subjected to column chromatography under the following conditions:

Absorbent: silica gel with a particle size of 0.04—0.063 mm.

Column: 20 mm in diameter, 100 mm in height; in dichloromethane.

Dissolving and washing: dichloromethane, 15 + 50 ml.

Development: with 50 ml of a mixture containing 1% of methanol in dichloromethane.

Elution: with 100 ml of a mixture containing 3% of methanol in dichloromethane; with 200 ml of a mixture containing 5% of methanol in dichloromethane; with 200 mml of a mixture containing 7% of methanol in dichloromethane.

TLC: with a solvent system containing a mixture of methanol and dichloromethane in a volume ratio of 3:20.

By using the above process, 160 mg (65%) of the aimed product are obtained; m.p.: 158—162°C (amorphous);

$[\alpha]_{546} = +18°$ (c = 1, chloroform).

$C_{49}H_{60}N_4O_{10}$ (molecular weight: 864).

IR (KBr, $cm^{-1}$): 740, 1020, 1140, 1220—1260, 1470, 1505, 1620, 1730, 2920, 3400.

**Claims**

1. Furfuryl derivatives of vinblastine-type bis-indoles of the general formula

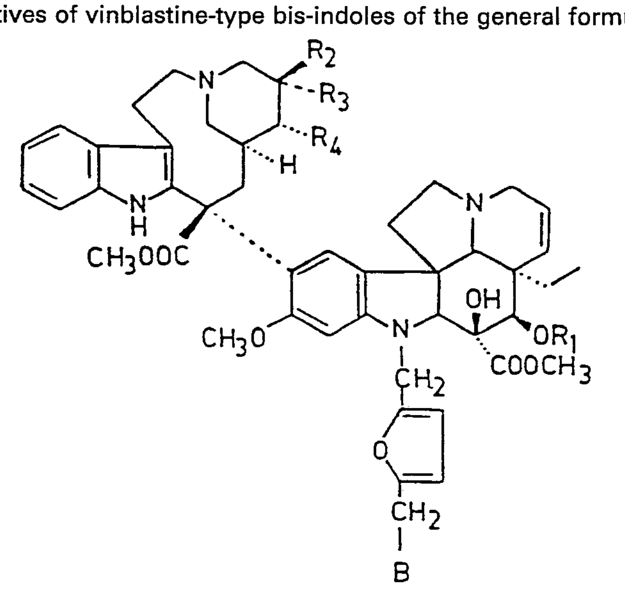

I,

wherein

$R_1$ stands for hydrogen atom or an acetyl group,

$R_2$ stands for a hydroxyl or ethyl group of β-position,

$R_3$ means an ethyl group of α-position,

$R_4$ represents a hydrogen or

$R_3$ and $R_4$ together represent an oxygen bridge and

B stands for a hydroxyl or an O-acyl group having 1 to 17 carbon atom(s),

as well as their acid addition salts.

2. Furfuryl derivatives of vinblastine-type according to claim 1, characterized in that the acyl group which is represented by B is such having from 1 to 10, particularly 1 to 4, carbon atom(s).

3. N-(Demethyl)-N-(4-hydroxymethylfurfuryl)-vinblastine, N-(demethyl)-N-(4-hydroxymethylfurfuryl)-leurosine, N-(demethyl)-N-(4-acetoxymethylfurfuryl)-leurosine and 17-(deacetyl)-N-(demethyl)-N-(4-hydroxymethylfurfuryl)-vinblastine as well as the hydrochlorides of these compounds.

4. A process for preparing the compounds according to claims 1 to 3, characterized in that

a) a compound of the general formula

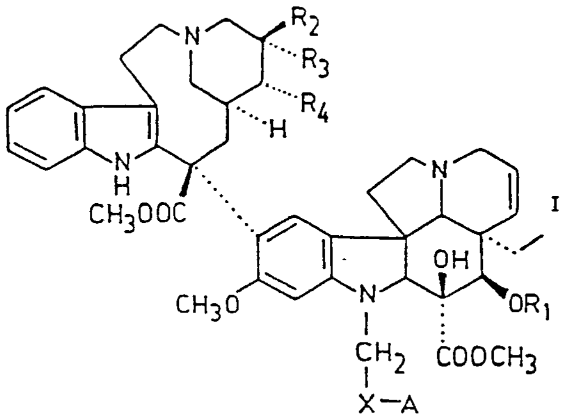

II,

wherein

$R_1$, $R_2$, $R_3$ and $R_4$ are the same as defined in claim 1

X means a sulphur or an oxygen atom;

and

A stands for an optionally substituted alkyl group having 1 to 10 carbon atom(s), phenylalkyl group having 1 to 10 carbon atom(s) in the alkyl part, cycloalkyl group having 3 to 8 carbon atoms, or a heteroaromatic group having 5 or 6 ring atoms,

is reacted with furfuryl alcohol in the presence of a mineral acid or a Lewis acid in a solvent, preferably in a halogenated hydrocarbon, then the reaction mixture is worked up and the desired product is isolated, and optionally

b) a compound of the general formula I, wherein

$R_1$ stands for an acetyl group, and

B represents a hydroxyl group

is transformed to a compound of formula I in which $R_1$ stands for a hydrogen atom or B represents an O-acyl group having from 1 to 17 carbon atom(s), respectively, by using a deacethylation or acylation, thus changing the meaning of $R_1$ and B, and then the thus obtained product is isolated and, if desired, the thus obtained product is transformed to an acid addition salt thereof.

5. Pharmaceutical compositions, characterized by a content of 1 or more compound(s) according to claims 1 to 3, as [an] active principle(s), suitably in admixture with 1 or more usual pharmaceutical carrier(s) and/or additive(s).

**Patentansprüche**

1. Furfurylderivate von Risindolen von Vinblastintypus, mit der allgemeinen Formel

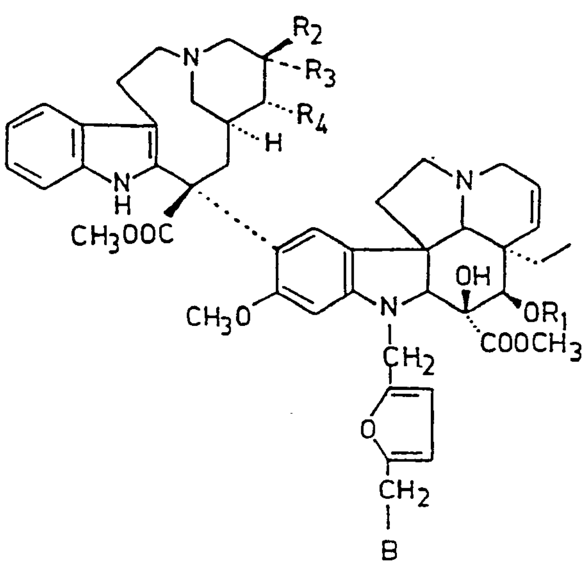

I,

in der

$R_1$ für ein Wasserstoffatom oder eine Acetylgruppe steht,
$R_2$ für eine Hydroxyl- oder Ethylgruppe in β-Stellung steht,
$R_3$ eine Ethylgruppe in α-Stellung bedeutet,
$R_4$ ein Wasserstoffatom darstellt oder
$R_3$ und $R_4$ zusammen eine Sauerstoffbrücke darstellen und
B für eine Hydroxyl- oder eine O-Acylgruppe mit 1 bis 17 Kohlenstoffatom(en) steht.

sowie deren Säureadditionssalze.

2. Furfurylderivate von Bisindolen vom Vinblastintypus nach Anspruch 1, dadurch gekennzeichnet, daß die durch B dargestellte Acylgruppe 1 bis 10, insbesondere 1 bis 4, Kohlenstoffatom(e) hat.

3. N-(Demethyl)-N-(4-hydroxymethylfurfuryl)-vinblastin, N-(Demethyl)-N-(4-hydroxymethylfurfuryl)-leurosin, N-(Demethyl)-N-(4-acetoxymethylfurfuryl)-leurosin und 17-(Deacetyl)-N-(demethyl)-N-(4-hydroxymethylfurfuryl)-vinblastin sowie die Hydrochloride dieser Verbindungen.

4. Verfahren zum Erzeugen der Verbindungen nach Anspruch 1 bis 3, dadurch gekennzeichnet, daß

a) eine Verbindung der allgemeinen Formel

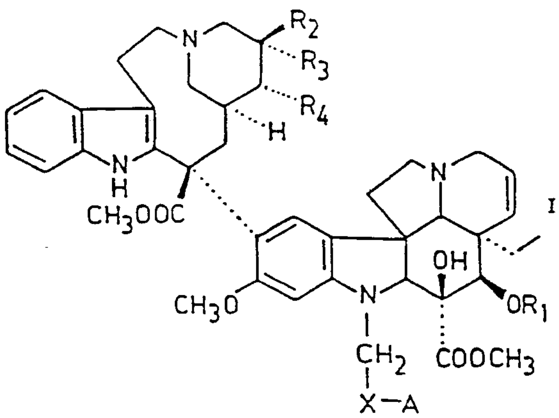

II,

in der

$R_1$, $R_2$, $R_3$ und $R_4$ dieselben Bedeutungen haben wie im Anspruch 1,
X ein Schwefel- oder ein Sauerstoffatom bedeutet und
A für eine gegebenenfalls substituierte Alkylgruppe mit 1 bis 10 Kohlenstoffatom(en), Phenylalkylgruppe mit 1 bis 10 Kohlenstoffatom(en) im Alkylteil, Cycloalkylgruppe mit 3 bis 8 Kohlenstoffatomen oder eine heteroaromatische Gruppe mit 5 oder 6 Ringatomen steht,

in einem Lösungsmittel, vorzugsweise in einem halogenierten Kohlenwasserstoff in Gegenwart einer Mineralsäure mit Furfurylalkohol umgesetzt wird und dann das Reaktionsgemisch aufgearbeitet und das gewünschte Produkt isoliert wird, und gegebenenfalls

b) eine Verbindung der allgemeinen Formel I, in der $R_1$ eine Acetylgruppe steht und
B eine Hydroxylgruppe darstellt,

durch Deacetylieren oder Acylieren in eine Verbindung der Formel I umgewandelt wird, in der $R_1$ für ein Wasserstoff steht oder B eine O-Acylgruppe mit 1 bis 17 Kohlenstoffatom(en) darstellt, so daß die Bedeutung von $R_1$ und B verändert wird, danach das so erhaltene Produkt isoliert wird und gegenbenenfalls das so erhaltene Produkt in ein Säureadditionssalz dieses Produktes umgewandelt wird.

5. Pharmazeutische Zusammensetzungen gekennzeichnet durch einen Gehalt an einer oder mehreren Verbindung(en) nach Anspruch 1 bis 3 als (einem) Wirkstoff(en), zweckmäßig im Gemisch mit einem oder mehreren üblichen pharmazeutischen Träger(n) und/oder Zusatzstoff(en).

**Revendications**

1. Dérivés furfuryle de bis-indoles du type vinblastine de formule générale:

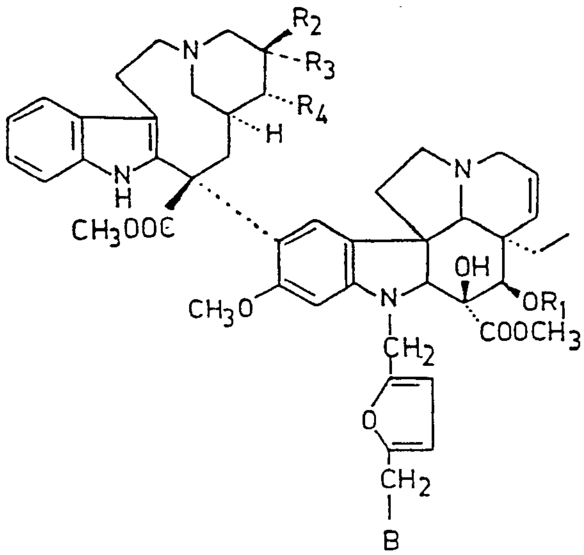

I,

dans laquelle:

$R_1$ représente un atome d'hydrogène ou un groupe acétyle,

$R_2$ représente un groupe hydroxy ou éthyle en position β,

$R_3$ représente un groupe éthyle en position α,

$R_4$ représente un atome d'hydrogène, ou

$R_3$ et $R_4$ représentent ensemble un pont oxygène et

B représente un groupe hydroxy ou O-acyle ayant 1 à 17 atomes de carbone,

ainsi que leurs sels d'addition acides.

2. Dérivés furfuryle de type vinblastine suivant la revendication 1, caractérisé en ce que le groupe acyle qui est représenté par B comprend 1 à 10, en particulier 1 à 4 atomes de carbone.

3. Dérivés furfuryle de type vinblastine suivant la revendication 1 ou 2, caractérisés en ce qu'il s'agit de la N-déméthyl-N-(4-hydroxyméthylfurfuryl)-vinblastine, la N-déméthyl-N-(4-hydroxyméthylfurfuryl)-leurosine, la N-déméthyl-N-(4-acétoxyméthylfurfuryl)-leurosine et de la 17-désacétyl-N-déméthyl-N-(4-hydroxyméthylfurfuryl)-vinblastine, ainsi que de leurs chlorhydrates.

4. Procédé de préparation des composés suivant les revendications 1 à 3, caractérisé en ce que:

a) un composé de formule générale:

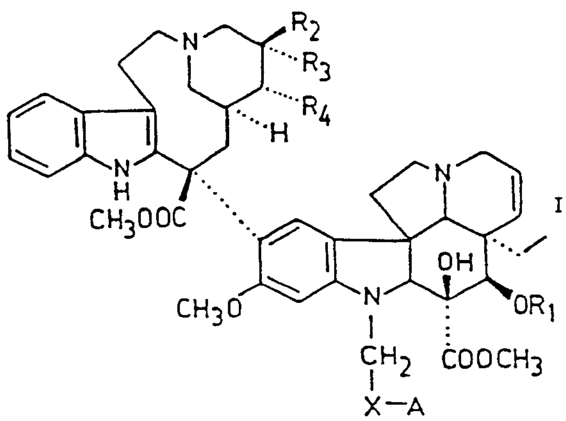

II,

dans laquelle

$R_1$, $R_2$, $R_3$ et $R_4$ sont tel que définis dans la revendication 1,

X est un atome de soufre ou d'oxygène et

A est un groupe alkyle éventuellement substitué ayant 1 à 10 atomes de carbone, un groupe phénylalkyle ayant 1 à 10 atomes de carbone dans la partie alkyle, un groupe cycloalkyle ayant 3 à 8 atomes de carbone ou un groupe hétéroaromatique ayant 5 ou 6 atomes dans le cycle,

est traité avec de l'alcool furfurylique en présence d'un acide minéral ou d'un acide de Lewis dans un solvant, de préférence un hydrocarbone halogéné, puis le mélange réactionnel est travaillé et le produit désiré est isolé, et éventuellement

b) un composé de formule générale 1, dans laquelle

$R_1$ représente un groupe acétyle et

B représente un groupe hydroxy,

est transformé en un composé de formule I dans laquelle $R_1$ est un atome d'hydrogène ou B est un groupe O-acyle ayant 1 à 17 atomes de carbone, respectivement, par désacétylation ou acylation, de façon à changer ainsi la signification de $R_1$ et de B, puis le produit désire est isolé,
et, si cela est désiré, le produit ainsi obtenu est converti en l'un de ses sels d'addition acides.

5. Compositions pharmaceutiques, caractérisées en ce qu'elles comprennent un ou plusieurs composés suivant les revendications 1 à 3 en tant que compostants actifs, convenablement en mélange avec un ou plusieurs supports et/ou additifs pharmaceutiques usuels.